# EUROPEAN PATENT SPECIFICATION

(11) **EP 3 206 590 B1**
(45) Date of publication and mention of the grant of the patent: **16.11.2022**
(21) Application number: 15804238.2
(22) Date of filing: 15.10.2015
(51) Int. Cl.: A61B 17/00, A61B 17/12, A61B 17/3207, A61B 17/221

(54) **CATHETER COMPRISING A CUTTING ELEMENT**
KATHETER MIT EINEM SCHNEIDELEMENT
CATHÉTER COMPRENANT UN ÉLÉMENT DE COUPE

(30) Priority: 17.10.2014 PL 40982414
(43) Date of publication of application: 23.08.2017
(73) Proprietor: Balton Sp. z o.o., 00-496 Warszawa (PL)
(72) Inventor: PLOWIECKI, Emil, 00-496 Warszawa (PL); HURKALA, Leszek, 00-496 Warszawa (PL)
(74) Representative: Orlinska, Dorota Irena
(86) International application number: PCT/PL2015/000167
(87) International publication number: WO 2016/060575

(56) References cited:
- WO-A1-2009/109967
- WO-A2-2004/112569
- US-A- 5 011 489
- US-A1- 2012 059 309
- US-A1- 2012 109 191

## Description

The object of the present invention is a catheter comprising a cutting element, i.e. an element that can controllably cut (damage) internal areas of walls of a blood vessel.

More particularly, the invention relates to the field of medical devices introduced percutaneously into a blood vessel in order to perform the procedure of its mechanical or chemical and mechanical obliteration (ablation, closure).

Chronic venous insufficiency is a peripheral vascular disease observed in the half of the adult population and leads to the formation of varicose veins, so-called "spider veins", venous inflammations, oedemas. Circulatory insufficiency in the lower extremities is a disease whose incidence increases with age, which results in an increasing demand for less invasive, non-surgical treatments to eliminate problems such as varicose veins of the lower extremities.

Venous stasis is a disorder caused by abnormal blood flow from the lower extremities to the heart. Contrary to appearances, not only the heart is responsible for ensuring the proper blood circulation in the organism. The work of the leg muscles (muscle pump) is responsible for returning blood to the heart. In a healthy body, muscles allow blood to return to the heart through the venous valve system. When lesions are observed, the "muscle pump" in the area of the extremities fails to work and / or the valve system does not sufficiently support the return of blood to the heart.

Methods of treating venous insufficiency (varicose veins) are limited. Currently used methods are based on stripping or ligation of insufficient venous sections, vein ablation using a laser or by the application of ultrasound, or steam, as well as through chemical obliteration by local injection of chemical substances that cause irritation to the inner walls of veins and, as a result, their coalescence (closing).

These methods have certain disadvantages, for example they may cause skin inflammations, vascular perforations, skin lesions or secondary venous patency (recanalisation). Furthermore, there are observed side effects related to the effects of the mentioned factors on blood cells and sometimes on adjacent, healthy tissues, including blood vessels, e.g. in the laser method causes overheating of the area surrounding the place of procedure, and also possible is the formation of clots that cause blockages in other areas of the patient's body.

WO 2004/112569 A2 discloses a vascular ablation apparatus that delivers a sclerosing solution during the disruption and/ or irritation of a vessel wall.

The present invention solves many of the problems indicated above by providing a simple and effective mechanical catheter for closing veins.

There is known the phenomenon that mechanical irritation to the inner walls of the vein causes its contractions. Sometimes, not very skilful use of intravascular medical equipment may cause contraction of the vessel. In the area where the vessel endothelium is mechanically damaged, cut, scratched or injured, a self-acting reaction of contraction (tightening) of the vessel is caused.

Within the present invention, a catheter provided with technical means for mechanical irritation of the vessels is proposed. Such a device allows solving many of the problems signalled above. Moreover, the developed catheter allows simultaneous support of mechanical obliteration by chemical obliteration, i.e. the catheter is provided with a channel for the administration of active substances. At the same time, the proposed solution helps to minimise the size of the catheter, which allows it to operate in narrow, deformed, affected vessels.

The object of the invention is a catheter as claimed in claim 1. Further embodiments of the invention are defined by the dependent claims as explained below. Methods do not form part of the claimed subject matter.

The assembly of the inner tube comprises the distal tube of a smaller diameter and the body tube of a greater diameter, forming the proximal portion of the assembly, wherein the cutting element is mounted on the distal tube.

The sleeve has a diameter equal to the diameter of the body tube of the assembly of the inner tube and this sleeve, from its proximal side, is immediately adjacent to the body tube.

The cutting element comprises 3 to 10 arms, which are preferably arc-shaped outward with respect to the axis of the catheter, and in the released state, in the vicinity of the sleeve, these arms are arranged at an acute angle in relation to the axis of the catheter.

The sharp endings of the cutting element are bent away from the arms outwardly with respect to the axis of the catheter, and these endings are conically pointed or form an elongated blade.

Along the catheter, the channel for the guidewire is led.

The catheter comprises a channel for fluid supply.

The cutting element is made of a single tube segment.

The insertion tube has an outer diameter of less than 2.2 mm.

The degree of release and unfolding of the arms of the cutting element is adjusted with the position of the distal edge of the insertion tube in relation to this element.

The cutting element has a shape adapted for repeated and multiple insertion of it entirely within the insertion tube, which in this position is in contact with the tip.

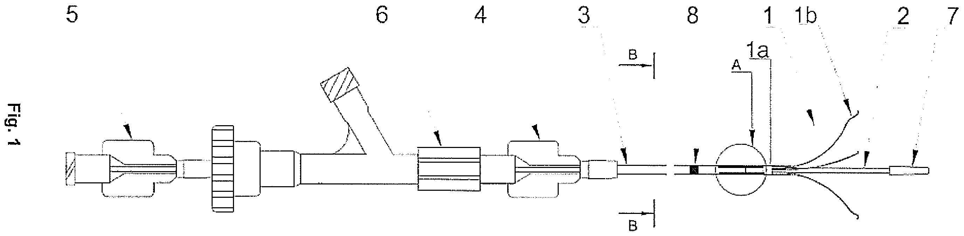

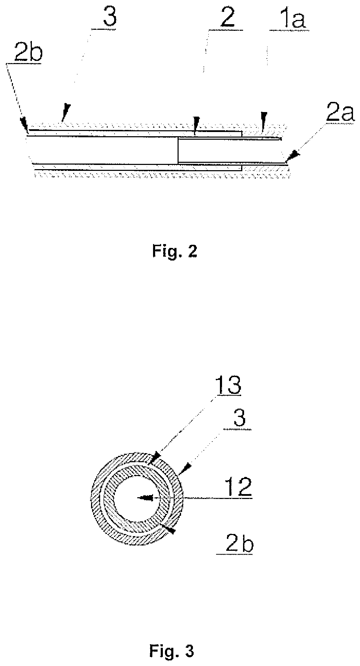

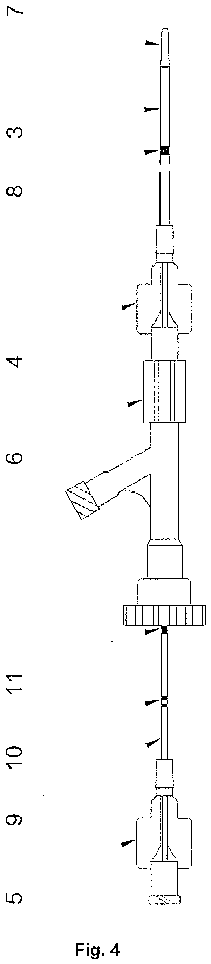

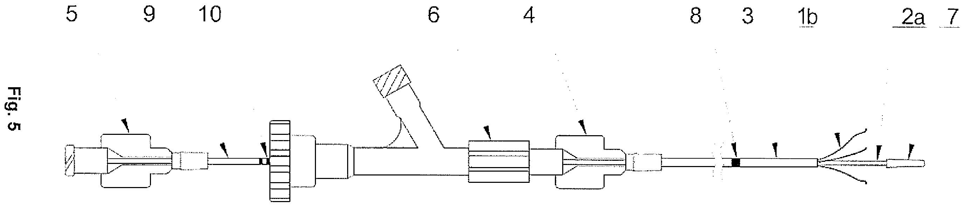

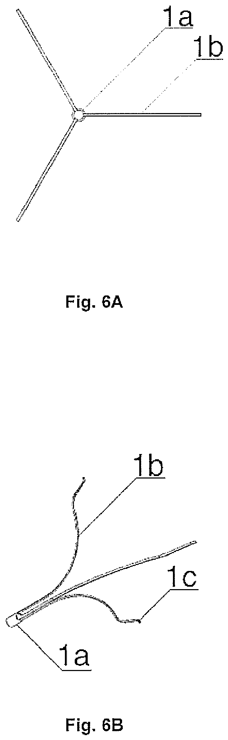

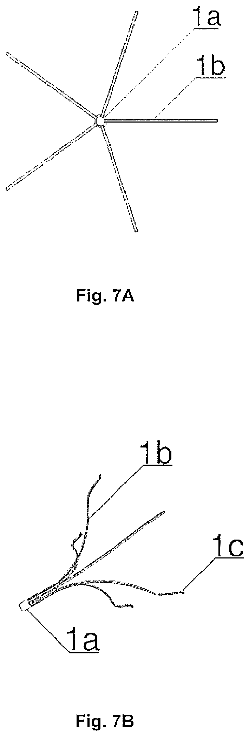

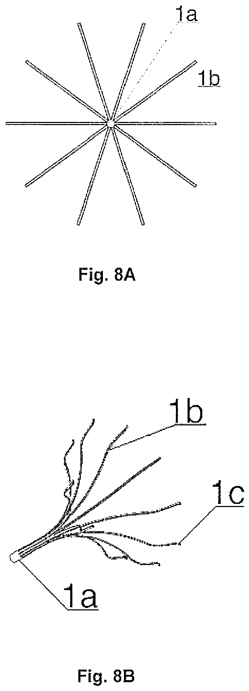

The object of the present invention in an exemplary embodiment is illustrated in the drawing, in which Fig. 1 shows a general view of the catheter with the cutting element completely released from the insertion tube, with the detail "A" and the plane of the cross section B-B also marked, Fig. 2 shows an enlarged detail "A" marked in Fig. 1 showing the point of connection of the body tube and the distal tube, as well as the place of mounting the sleeve of the cutting element, and Fig. 3 shows an enlarged B-B cross-sectional view of the catheter in the place marked on the Fig. 1, Fig. 4 shows the catheter of Fig. 1 in the closed position, with the cutting element hidden under the insertion tube, Fig. 5 shows the catheter of Fig. 1 and 4 in an intermediate position, with a partially extended cutting element, which is partially overlapped by the insertion tube, Fig. 6A and 6B, 7A and 7B, and 8A and 8B show an enlarged structure of the cutting element with, respectively, three, five and ten arms, wherein Fig. 6A, 7A and 8A show a view of the cutting elements exploded (open) from the side of their sharp ends, and Fig. 6B, 7B and 8B show a general view of these cutting elements in an enlargement.

The catheter according to the present invention comprises a springy resilient (elastic) cutting element that is mounted on the distal tube **2a** of the assembly **2** of the inner tube, and comprises, successively, viewed from the proximal (the operator's) side, the sleeve **1a** and the arc-curved longitudinal arms **1b** with the properly formed and bent outwards sharpened endings **1c**, connected thereto at its distal edge and facing towards the atraumatic tip **7.** The arms **1b** form a band of identical elements (branches of the cutting element protruding from the axis of the catheter). It is possible to profile the arms 1**b** in any way in order to achieve the effect of irritation of the vessel wall by the sharp endings **1c.** The assembly **2** of the inner tube consists of two concentrically arranged tubes, the distal tube **2a** and the body tube **2b** of a larger diameter, which is the proximal portion of the assembly **2** of the inner tube. The distal tube **2a** is adapted in its diameter to the body tube **2b,** so that it is possible to contiguously mount the tubes one in another at the point of their connection (shown in Fig. 2). The body tube **2b** supports the tube **2a** with a smaller diameter, located further, in the distal portion of the catheter. The sleeve **1a** of the cutting element **1** has a diameter equal (or nearly equal) to the diameter of the body tube **2b.** The described mutual arrangement of the tubes **2a** and **2b** and the sleeve **1a** is shown in Fig. 2. The insertion tube **3,** with a diameter larger than the tube **2a** and the sleeve **1a,** covers both these elements, together with the arms **1b** and the endings **1c** of the cutting element, and therefore the cutting element **1** without obstacles can be repeatedly released and retracted by sliding it within the tube **3.** The insertion tube **3** has preferably an outer diameter of less than 2.2 mm. Preferably, the sleeve **1a** is mounted on the distal tube **2a** in such a manner that the proximal edge of the sleeve **1a** is in direct contact with the distal edge of the body tube **2b.**

Number of the arms **1b** of the cutting element is in the range of 3 to 10, e.g. the cutting element can comprise 3, 4, 5, 6 arms **1b.**

Preferably, the arms **1b** in relation to the axis of the sleeve **1a** and the axis of the catheter are arranged symmetrically e.g. on the circumference of the sleeve at every 120° (3 arms) or every 90° (4 arms), etc., as shown demonstratively in Fig. 6A, 7A and 8A. The arms **1b** have the form of branches extending from the sleeve **1a** and can have a circular cross-section (particularly when formed from wires attached to the sleeve) or approximately trapezoidal cross-section (when formed by cutting out the arms and the sleeve from one section of metal tube). The arms **1b** are formed so that when releasing the element **1** from the catheter they radially extend outward, away from the assembly **2** of the inner tube towards the walls of the blood vessel. The arms **1b** after full extension of the cutting element (releasing it from the insertion tube **3**) are arranged in a section adjacent to the sleeve at an acute angle in relation to the axis of the catheter, e.g. at an angle of 60° or 45°. The arms **1b** of the end portion, remote from the sleeve **1a,** are profiled (bent) arcuately, so that the sharp endings **1c** are adjacent to the walls of the vessel. The arms **1b** can also be formed as a line similar to the letter "S", or in a yet different way, however, the endings **1c** are always most remote from the axis of the catheter. Preferably, the sharp endings **1c** are bent away from the arms **1b** outwardly with respect to the axis of the catheter, i.e. they are more strongly curved outwardly than the line of curvature of the arms **1b.** The endings **1c** can be conically pointed or can create slightly elongated blade by flattening the end sections of the arms **1b.**

The cutting element **1** can be completely cut out from the section of the tube of metal or other material and then the beginning of such a tube is not treated (the sleeve remains) and in the remaining portion of the tube cutting is carried out to obtain a desired number of arms, therefore 3 to 10 arms are cut in the tube. It is also possible to prepare the cutting element **1** by mounting previously prepared single arms **1b** to the section of the tube (the sleeve). These arms are mounted to the sleeve in such manner that the diameter of the retracted cutting element **1** along its length does not exceed the diameter of the sleeve **1a,** when the element **1** is retracted in the insertion tube **3.** As a result, the catheter can have a minimum diameter limited only with the diameter of the sleeve **1a,** increased by the addition of the insertion tube **3.** Between the insertion tube **3** and the assembly **2** of the inner tube the space is maintained constituting the channel **13** for fluid supply, for example of sclerotisation chemicals (including foams), pharmacologically active agents, saline solution, etc. Inside the assembly **2** of the inner tube, along the entire catheter, the channel **12** for the guidewire was led, preferably for the guidewire of the dimension 0.89 mm (0.035").

The guidewire provides stable positioning of the catheter during the procedure.

In its proximal portion, the catheter comprises known in the art elements accessible for the operator, enabling control of the device. Subsequently, from the proximal side, the catheter is provided with the cap **5** of the assembly of the inner tube, from which the cuff **9** of the assembly of the inner tube is led, next the "Y" cap **6** and the cap **4** of the insertion tube. The cap **4** and the "Y" cap **6** are used together to control the insertion tube.

On the distal tube **2a,** at its end, after the cutting element **1,** there is mounted the atraumatic soft tip **7** enabling atraumatic insertion of the catheter into the vessel. The catheter can preferably comprise markers visible through ultrasound and / or X-ray imaging, enabling operation of the device during procedure. On the insertion tube **3,** at its distal portion, there is provided the marker **8,** and on the body tube **2b,** at its proximal side, there is provided the first marker **10** followed by the second marker **11.** Along the entire catheter there is led the internal channel **12** for the guidewire, shown as the middle space in Fig. 3.

The catheter is inserted into the blood vessel within the guidewire previously positioned in said vessel, through a typical shrink in the position where the insertion tube **3** covers the entire retracted cutting element **1** (the arms **1b** and the endings **1c** then adhere to the distal tube **2a**). The insertion tube **3** comes then to the soft tip **7** - the catheter is closed. Full extension of the cutting element **1** occurs when pushing the cap **5** all the way to the "Y" cap **6.**

To lock the position of the assembly **2** of the inner tube in relation to the insertion tube **3** one needs to tighten the nut on the "Y" cap **6.** Tightening the nut seals the space between the insertion tube **3** and the assembly **2** of the inner tube, and thus venting of the channel **13** shown in Fig. 3 is possible. At the distal portion of the insertion tube **3** there is applied the marker **8,** which after extension off the shrink during procedure means that the cutting element **1** is now pushed all the way to the shrink. At the proximal portion of the assembly **2** of the inner tube there are applied two markers. The marker **11** means complete retraction of the cutting element **1** in the catheter, the marker **10** means partial extension of the cutting element **1.**

Fig. 5 shows the cutting element **1** partially extended (released) from the insertion tube **3.** In such a position, the cutting element can also fulfil its function, i.e. the sharp ends **1c** are in contact with the inner wall of the vessel and cause its longitudinal incisions when moving the catheter. The described construction of the catheter thus does not require the use of a completely extended cutting element **1** during the procedure. Depending on the structure, size and shape of the affected vessel, the operator can smoothly grade the folding of the arms **1b,** i.e. adjust the diameter of the extended cutting element to the diameter of the vessel by adjusting the position of the insertion tube **3** in relation to the element **1.** The "Y" cap **6** enables locking the cutting element at a predetermined position.

The above described construction of the catheter allows smooth and fast multiple repeats of the procedure of incision of the vessel, i.e. its mechanical sclerotisation/obliteration. The cutting element **1** is susceptible to reproducible, multiple insertions of it entirely within insertion tube **3.** After the insertion of the catheter and release of the cutting element **1** from the insertion tube **3,** it is moved with a uniform motion in the reverse direction (back to the operator), which causes longitudinal incision (irritation, scratch) of the vessel endothelium along the required length of the sclerotised vein. Then the cutting element **1** can be retracted in the insertion tube **3** (still intravascularly), moved in this closed position again to a remote (from the operator) portion of the vessel, and then released again, so that the process of incision of the vessel is repeated on the same or another portion of the vessel. As a result of irritation of the vessel there are almost immediately caused contractions of the vessel and its closure.

The procedure using the catheter according to the present invention can be carried out also as a mechanical and chemical sclerotisation. In such a case the operations described above are repeated, but through the side channel of the "Y" cap **6** there is additionally administered a dose of sclerotisation agent in the form of a liquid solution or a foam prepared using the Tessari method. The catherer is pulled (withdrawn) with a uniform motion, cutting the endothelium of the vein with the cutting elements **1** while injecting sclerotisation agent. As before, the operation should be performed over the entire length of sclerotised vein. Mechanical and chemical sclerotisation of the same vein can be performed repeatedly. The invention is defined by the claims.

The marks on the drawings:
- 1: cutting element
- 1a: sleeve of the cutting element
- 1b: arm / arms of the cutting element
- 1c: sharp ending of the arm
- 2: assembly of the inner tube
- 2a: distal tube
- 2b: body tube
- 3: insertion tube
- 4: cap of the insertion tube
- 5: cap of the assembly of the inner tube
- 6: "Y" cap
- 7: soft tip
- 8: marker of the insertion tube
- 9: cuff of the assembly of the inner tube
- 10: first marker of the body tube
- 11: second marker of the body tube
- 12: channel for the guidewire
- 13: channel for fluid supply

## Claims

1. An obliteration catheter to controllably cut or damage internal areas of walls of a blood vessel comprising a cutting element, an insertion tube (**3**) and an inner tube, wherein said cutting element comprises a springy resilient cutting element (**1**) consisting of a sleeve (**1a**) and profiled longitudinal arms (**1b**) with sharp endings (**1c**), whereas said longitudinal arms (**1b**) protrude from said sleeve (**1a**), **characterised in that** said inner tube has a distal atraumatic tip (**7**), wherein said longitudinal arms (**1b**) are directed towards said atraumatic tip (**7**), wherein the cutting element (**1**) is mounted on an assembly (**2**) of the inner tube, proximally from said atraumatic distal tip (**7**).

2. The catheter according to claim 1, **characterised in that** the assembly (**2**) of the inner tube comprises a distal tube (**2a**) with a smaller diameter and a body tube (**2b**) with a larger diameter, constituting the proximal portion of the assembly (**2**) of the inner tube, wherein the cutting element (**1**) is mounted on the distal tube (**2a**).

3. The catheter according to claim 2, **characterised in that** the sleeve (**1a**) has a diameter equal to the diameter of the body tube (**2b**) of the assembly (**2**) of the inner tube.

4. The catheter according to claim 2 or 3, **characterised in that** the sleeve (**1a**) at its proximal side is immediately adjacent to the body tube (**2b**).

5. The catheter according to any one of claims. 1 to 4, **characterised in that** the cutting element (**1**) comprises 3 to 10 longitudinal arms (**1b**).

6. The catheter according to any one of claims 1 to 5, **characterised in that** the cutting element (**1**) has the longitudinal arms (**1b**) arc-shaped outwardly with respect to the axis of the catheter, and in the released state, in the vicinity of the sleeve (**1a**), the longitudinal arms (**1b**) are arranged at an acute angle in relation to the axis of the catheter.

7. The catheter according to any one of claims 1 to 6, **characterised in that** the sharp endings (**1c**) are bent away from the longitudinal arms (**1b**) outwardly in relation to the axis of the catheter, and the sharp endings (**1c**) are conically pointed or are in the form of elongated blades made by flattening end sections of the longitudinal arms (**1b**).

8. The catheter according to any one of claims 1 to 7, **characterised in that** inside the assembly (**2**) of the inner tube, along the catheter there, a channel (**12**) of the guidewire is led.

9. The catheter according to any one of claims 1 to 8, **characterised in that** between the insertion tube (**3**) and the assembly (**2**) of the inner tube the catheter comprises a channel (**13**) for fluid supply.

10. The catheter according to any one of claims 1 to 9, **characterised in that** the cutting element (**1**) is made from a single tube segment.

11. The catheter according to any one of claims 1 to 10, **characterised in that** the insertion tube (**3**) has an outer diameter of less than 2.2 mm.

12. The catheter according to any one of claims 1 to 11, **characterised in that** the degree of release and unfolding of the longitudinal arms (**1b**) of the cutting element (**1**) is adjusted with the position of the distal edge of the insertion tube (**3**) with respect to the cutting element (**1**).

13. The catheter according to any one of claims 1 to 12, **characterised in that** the cutting element (**1**) has a shape adapted for repeated and multiple insertion of it entirely within the insertion tube (**3**), which in this position is in contact with the atraumatic tip (**7**).

## Patentansprüche

1. Verödungskatheter zum kontrollierten Schneiden oder Beschädigen innerer Bereiche von Wänden eines Blutgefäßes mit einem Schneideelement, einem Einführungsrohr (3) und einem Innenrohr, wobei das Schneideelement ein federnd elastisches Schneideelement (1) umfasst, das aus einer Hülse (1a) und profilierten Längsarmen (1b) mit scharfen Enden (1c) besteht, wobei die Längsarme (1b) aus der Hülse (1a) herausragen, **dadurch gekennzeichnet, dass** das Innenrohr eine distale atraumatische Spitze (7) aufweist, wobei die Längsarme (1b) auf die atraumatische Spitze (7) gerichtet sind, wobei das Schneidelement (1) an einer Baugruppe (2) des Innenrohrs proximal von der atraumatischen distalen Spitze (7) angebracht ist.

2. Katheter nach Anspruch 1, **dadurch gekennzeichnet, dass** die Baugruppe (2) des Innenrohrs ein distales Rohr (2a) mit einem kleineren Durchmesser und ein Körperrohr (2b) mit einem größeren Durchmesser umfasst, die den proximalen Teil der Baugruppe (2) des Innenrohrs bilden, wobei das Schneidelement (1) auf dem distalen Rohr (2a) angebracht ist.

3. Katheter nach Anspruch 2, **dadurch gekennzeichnet, dass** die Hülse (1a) einen Durchmesser aufweist, der dem Durchmesser des Körperrohrs (2b) der Baugruppe (2) des Innenrohrs entspricht.

4. Katheter nach Anspruch 2 oder 3, **dadurch gekennzeichnet, dass** die Hülse (1a) an ihrer proximalen Seite unmittelbar an das Körperrohr (2b) angrenzt.

5. Katheter nach einem der Ansprüche 1 bis 4, **dadurch gekennzeichnet, dass** das Schneidelement (1) 3 bis 10 Längsarme (1b) aufweist.

6. Katheter nach einem der Ansprüche 1 bis 5, **dadurch gekennzeichnet, dass** das Schneidelement (1) die Längsarme (1b) bogenförmig nach außen zur Katheterachse aufweist und die Längsarme (1b) im freigegebenen Zustand in der Nähe der Hülse (1a) in einem spitzen Winkel zur Katheterachse angeordnet sind.

7. Katheter nach einem der Ansprüche 1 bis 6, **dadurch gekennzeichnet, dass** die scharfen Enden (1c) von den Längsarme (1b) nach außen in Bezug auf die Achse des Katheters abgebogen sind und die scharfen Enden (1c) konisch zugespitzt sind oder die Form länglicher Klingen haben, die durch Abflachen von Endabschnitten der Längsarme (1b) hergestellt sind.

8. Katheter nach einem der Ansprüche 1 bis 7, **dadurch gekennzeichnet, dass** im Inneren der Baugruppe (2) des Innenrohrs entlang des Katheters ein Kanal (12) des Führungsdrahtes geführt ist.

9. Katheter nach einem der Ansprüche 1 bis 8, **dadurch gekennzeichnet, dass** der Katheter zwischen dem Einführungsrohr (3) und der Baugruppe (2) des Innenrohrs einen Kanal (13) für die Flüssigkeitszufuhr aufweist.

10. Katheter nach einem der Ansprüche 1 bis 9, **dadurch gekennzeichnet, dass** das Schneidelement (1) aus einem einzigen Schlauchsegment gefertigt ist.

11. Katheter nach einem der Ansprüche 1 bis 10, **dadurch gekennzeichnet, dass** das Einführungsrohr (3) einen Außendurchmesser von weniger als 2,2 mm aufweist.

12. Katheter nach einem der Ansprüche 1 bis 11, **dadurch gekennzeichnet, dass** der Grad der Freigabe und Entfaltung der Längsarme (1b) des Schneidelements (1) mit der Position der distalen Kante des Einführungsrohrs (3) in Bezug auf das Schneidelement (1) eingestellt wird.

13. Katheter nach einem der Ansprüche 1 bis 12, **dadurch gekennzeichnet, dass** das Schneidelement (1) eine Form aufweist, die ein wiederholtes und mehrfaches Einführen desselben vollständig in das Einführungsrohr (3) ermöglicht, das in dieser Position mit der atraumatischen Spitze (7) in Kontakt ist.

## Revendications

1. Un cathéter d'oblitération pour couper ou endommager de manière contrôlable les zones internes des parois d'un vaisseau sanguin comprenant un élément de coupe, un tube d'insertion (3) et un tube interne, dans lequel ledit élément de coupe comprend un élément de coupe résilient élastique (1) consistant en un manchon (1a) et des bras longitudinaux profilés (1b) avec des extrémités pointues (1c), tandis que lesdits bras longitudinaux (1b) font saillie dudit manchon (1a), **caractérisé en ce que** ledit tube interne a une pointe atraumatique distale (7), dans lequel lesdits bras longitudinaux (1b) sont dirigés vers ladite pointe atraumatique (7), dans lequel l'élément de coupe (1) est monté sur un ensemble (2) du tube interne, à proximité de ladite pointe distale atraumatique (7).

2. Un cathéter selon la revendication 1, **caractérisé en ce que** l'ensemble (2) du tube interne comprend un tube distal (2a) de plus petit diamètre et un tube de corps (2b) de plus grand diamètre, constituant la partie proximale de l'ensemble (2) du tube interne, dans lequel l'élément de coupe (1) est monté sur le tube distal (2a).

3. Le cathéter selon la revendication 2, **caractérisé en ce que** le manchon (1a) a un diamètre égal au diamètre du tube de corps (2b) de l'ensemble (2) du tube interne.

4. Le cathéter selon la revendication 2 ou 3, **caractérisé en ce que** le manchon (1a), sur son côté proximal, est immédiatement adjacent au tube de corps (2b).

5. Le cathéter selon l'une quelconque des revendications 1 à 4, **caractérisé en ce que** l'élément de coupe (1) comprend 3 à 10 bras longitudinaux (1b).

6. Le cathéter selon l'une quelconque des revendications 1 à 5, **caractérisé en ce que** l'élément de coupe (1) présente les bras longitudinaux (1b) en forme d'arc vers l'extérieur par rapport à l'axe du cathéter, et à l'état relâché, à proximité du manchon (1a), les bras longitudinaux (1b) sont disposés selon un angle aigu par rapport à l'axe du cathéter.

7. Le cathéter selon l'une quelconque des revendications 1 à 6, **caractérisé en ce que** les extrémités pointues (1c) sont recourbées à l'écart des bras longitudinaux (1b) vers l'extérieur par rapport à l'axe du cathéter, et les extrémités pointues (1c) sont coniquement pointues ou ont la forme de lames allongées réalisées par aplatissement des sections d'extrémité des bras longitudinaux (1b).

8. Le cathéter selon l'une quelconque des revendications 1 à 7, **caractérisé en ce qu'**à l'intérieur de l'ensemble (2) du tube interne, le long du cathéter, un canal (12) du fil de guidage est mené.

9. Le cathéter selon l'une quelconque des revendications 1 à 8, **caractérisé en ce qu'**entre le tube d'insertion (3) et l'ensemble (2) du tube interne, le cathéter comprend un canal (13) d'alimentation en fluide.

10. Le cathéter selon l'une quelconque des revendications 1 à 9, **caractérisé en ce que** l'élément de coupe (1) est réalisé à partir d'un seul segment de tube.

11. Le cathéter selon l'une quelconque des revendications 1 à 10, **caractérisé en ce que** le tube d'insertion (3) a un diamètre extérieur inférieur à 2,2 mm.

12. Le cathéter selon l'une quelconque des revendications 1 à 11, **caractérisé en ce que** le degré de relâchement et de déploiement des bras longitudinaux (1b) de l'élément de coupe (1) est ajusté avec la position du bord distal du tube d'insertion (3) par rapport à l'élément de coupe (1).

13. Le cathéter selon l'une quelconque des revendications 1 à 12, **caractérisé en ce que** l'élément de coupe (1) a une forme adaptée pour une insertion répétée et multiple de celui-ci entièrement à l'intérieur du tube d'insertion (3) qui dans cette position est en contact avec la pointe atraumatique (7).
